# EUROPÄISCHE PATENTANMELDUNG

(11) **EP 1 175 876 A1**
(43) Veröffentlichungstag der Anmeldung: **30.01.2002**
(21) Anmeldenummer: 01118345.6
(22) Anmeldetag: 27.07.2001
(51) Int. Cl.: A61F 2/36

(54) **Schenkelhalserhaltende Prothese**

(30) Priorität: 29.07.2000 DE 10036986
(71) Anmelder: Draenert, Klaus, Prof.Dr.med., 81545 München (DE)
(72) Erfinder: Draenert, Klaus, 81545 München (DE); Bläsius, Klaus, 52224 Stolberg (DE)
(74) Vertreter: Wahl, Hendrik, Dr.-Ing.

(57) **Zusammenfassung**

Die vorliegende Erfindung betrifft im Wesentlichen einen Hüftgelenksendoprothesenstiel für die zementfreie oder zementierte Verankerung im Knochen, der sich im Schenkelhals und der proximalen Metaphyse verankert und die inneren den Oberschenkelknochen versteifenden Spongiosa- und Kompaktastrukturen erhält, den axialen Zugang zum Markkanal zum Konstruktions- element macht und parabelförmig gekrümmte Mantelflächen zur Optimierung der Kraftübertragung auf den Knochen besitzt.

## Beschreibung

### Hintergrund der Erfindung

Anatomisch bestehen wenig Korrelationen zwischen der Morphologie der Diaphyse und der Gestaltung und Ausladung der Metaphyse, es gelten jedoch die physiologischen Gesetze der Kraftübertragung, die besagen, dass der Knochen dort am stärksten versteift ist, wo seine Beanspruchung am größten ist. Die Beanspruchung kann in der Knochenstruktur abgelesen werden und diese Befunde können sich unter Berücksichtigung des Interfaces einer künstlichen Prothese im Design einer Prothese niederschlagen. Es wurde gefunden, dass nicht die Prothese die besten Langzeitergebnisse erwarten läßt, die den Knochen am besten in seiner kompakten Hülle ausfüllt (costume made), sondern vielmehr die, die die versteifenden Strukturen des Knochens optimal erhält (Draenert K et al. 1999 Manual of Cementing Technique Berlin Heidelberg : Springer). Es ist das Ziel dieser Erfindung, die versteifenden Strukturen im proximalen Femur zu erhalten, proximal Kraft in den Knochen einzuleiten und ein Maximum an Rotationsstabilität zu erreichen.

### Stand der Forschung

Herkömmliche Stiele von femoralen Komponenten folgen in ihrem Design einer Frontalprojektion des Femurknochens, sei es als zementierte Komponente oder als zemenfrei verankertes Design, so Charnley J (1960) Anchorage of the Femoral Head Prosthesis to the Shaft of the Femur J Bone and Joint Surg B42: 28-30, oder auch Zweymüller K A et al (1988) Biologic Fixation of a press-fit Titanium Hip Joint Endoprosthesis Clin Orthop 235, 195-206. Es hat sich jedoch gezeigt, dass ein wesentliches Belastungsmoment beim Aufsetzen der Ferse, beim Treppensteigen oder allgemein beim Strecken aus der Beugestellung des Hüftgelenkes auftritt. Das Studium der Entstehung des Schenkelhalses in der Phylo- und Ontogenese läßt klar erkennen, dass in dieser sogenannten Heel-Strike-Phase ein großes Retrotorsionsmoment auf den Schenkelhals einwirkt, welches es zu berücksichtigen gilt.

Bislang war es nur gelungen, mit Hilfe vom Knochenzement proximal in der Metaphyse Kraft einzuleiten, wie dies in Draenert K und Draenert Y 1992 Forschung und Fortbildung in der Chirurgie des Bewegungsapparates 3: Die Adaptation des Knochens an die Deformation durch Implantate München: Art and Science beschrieben ist. MAR Freeman fragte 1986, warum man den Schenkelhals reseziere ( Freeman MAR 1986 Why Resect the Neck J Bone Joint Surg 68B, 346-349 ); es wurde dabei auch die erhöhte Rotationsstabilität angesprochen, jedoch nicht in die Konstruktion umgesetzt, da die Bedeutung der anatomischen Strukturen noch nicht erkannt worden war. Dementsprechend war der daraus resultierende Stiel auch ein gerader Stiel ohne Rechts-Linkssymmetrie.

Es wurde jedoch gefunden, dass gerade die anatomischen Strukturen im proximalen Femur die Steifigkeit dieses Knochens und die hohe Biegebeanspruchbarkeit und Torsionsfestigkeit ausmachen; es sind gleichsam alle anatomischen Strukturen bis in die feinste Aussteifung durch die Spongiosa eine in sich verwrungene Gesamtkonstruktion, die zu erhalten ist.

Dieser Entdeckung wurde in der nachfolgenden Erfindung Rechnung getragen.

### Beschreibung der Erfindung

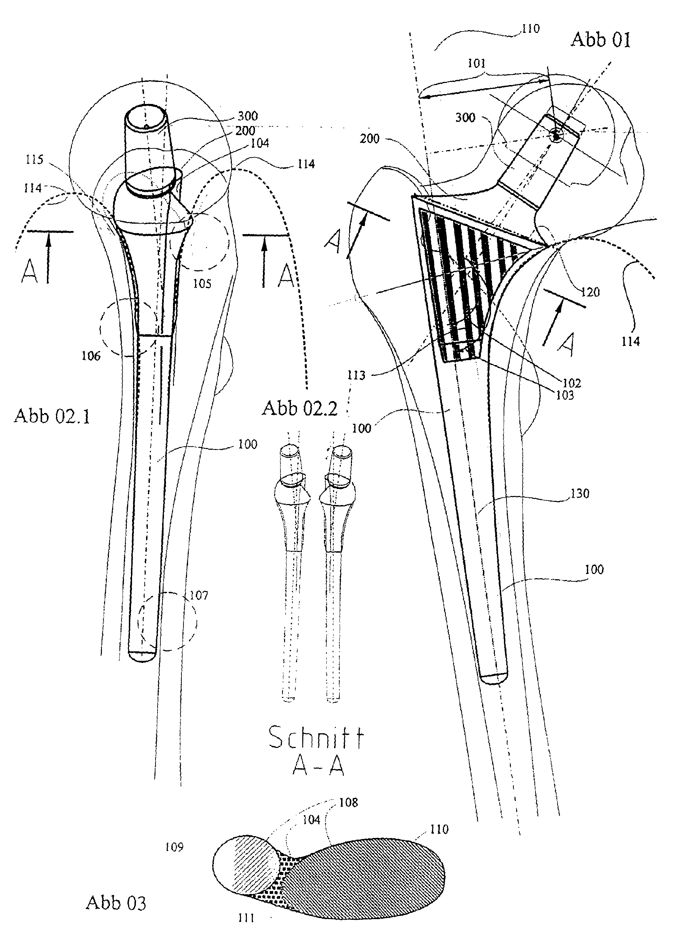

Ein wesentliches Element des Prothesenstieles ist die geradlinige Eröffnung des Markkanales durch das Trochantermajormassiv *(Abb. 01/110).* Die Osteotomie erhält den gesamten Schenkelhals *(Abb. 01/120).* Die Konstruktion des Stieles *(Abb. 01/100)* berücksichtigt, dass dieser dann geradlinig eingeschoben werden kann, wenn ein Führungsinstrument in dem Eröffnungskanal ohne Widerstand in den Markkanal eingeführt werden kann. Dies ist dann der Fall, wenn die Eröffnung korrekt entlang der Dorsalwand des Schenkelhalses in gerader Verlängerung der Kanalachse durch das Trochantermassiv durchgeführt worden war. Die Dorsalwand des Schenkelhalses bildet zur Dorsalwand des Markkanales eine gerade Linie.

Die Konstruktion der Prothese berücksichtigt diese Anatomie und respektiert einen Zylinder *(Abb. 03/109)* als Konstruktionselement um die Kanalachse *(Abb. 01/130)*, um den herum entsprechend der anatomischen Freiräume das Design des Stieles konstruiert wurde *(Abb.* *02.1)*, in welcher Weise sich spiegelsymmetrische Rechts-Linksversionen ergeben *(Abb. 02.2).*

Daraus ergibt sich ein spiegelsymmetrischer Querschnitt *(Abb.03)*, welcher als auffälligstes Merkmal über die Dorsalseite des Stieles eine flache bis tiefe Inzisur aufweist *(Abb 03/104)*, die geradlinig in die Spitze der Prothese ausläuft.

Insgesamt resultiert daraus eine konvex-konkav-konvexe Schwingung *(Abb 03/109 - Abb 03/104-Abb 03/110) =* S-förmige Schwingung *(Abb 03/108).*

Der Rekonstruktion des Rotationszentrums berücksichtigt zwei Parameter: Einmal wurde gefunden, dass eine enge Beziehung besteht zwischen der sogenannten Metaphysenöffnungsebene (Draenert et al. 1999 Manual of Cementing Technique Berlin Heidelberg: Springer ) und dem Rotationszentrum (RZ), in dem dieses in der Regel 25 mm oberhalb dieser Ebene liegt. Zum anderen lehrte die Erfahrung, dass das Offset einer Prothese, das ist der Abstand von der Konstruktionsachse oder Kanalachse zum RZ des Kopfes vom Femur, mit 45mm zu lang war (Charnley J 1989 Low Friction Arthroplasty of the Hip: Theory and Practice Berlin Heidelberg New York : Springer). Als optimal wurde aufgrund der klinischen Erfahrung ein Hebelarm von 40 mm Offset erachtet. Dieses liegt der Konstruktion zugrunde *(Abb. 01/101).*

Dieselbe Erfahrung aus der klinischen Praxis lehrt, dass ein physiologischer Centrum-Collum-Diaphysenwinkel CCD-Winkel von 126° zu einer erhöhten Versagensquote im Hinblick auf die Langzeitverankerung führt. Hier hat sich ein Winkel von 135° *(Abb. 01/102)* über viele Jahre bewährt, da die Kraft vermehrt als direkte Druckkraft in das Rohr des knöchernen Femurs eingeleitet wird. Nun ist es eine Eigenheit dieser Prothese, dass die Achse des Konus nicht identisch sein muss mit dem CCD -Winkel, sondern um 3° - 15°, in der Regel 5° - 6° *(Abb. 01/103)* steiler verlaufen kann, um diesen Effekt noch zu verstärken.

Die Länge des Stieles berücksichtigt die S-förmige Schwingung im seitlichen Strahlengang und liegt zwischen 14 cm und 22 cm, in der Regel um 15 cm, um eine sichere Verblockung zwischen diesen Krümmungen zu erreichen *(Abb. 02.1/105, Abb. 02.1/106* und *Abb. 02.1/107).*

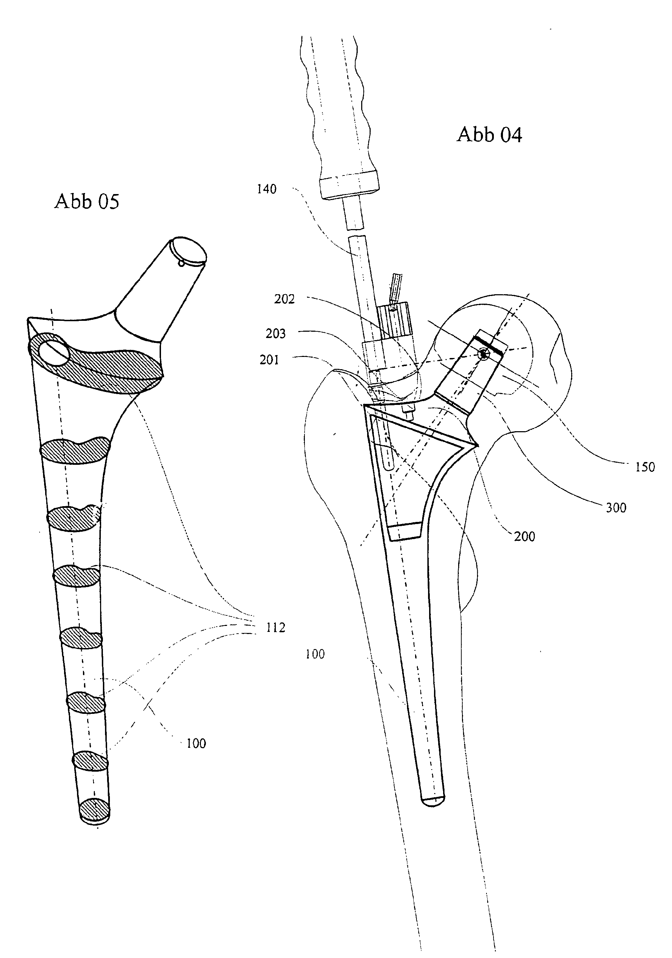

Das Besondere an dieser Prothese ist die geradlinige und doch anatomisch spiegelsymmetrische Konstruktion: die Dorsalmantelfläche ist wellig in Form einer gerundeten "3" mit ungleichen Schenkeln ausgebildet *(Abb. 03/108),* wodurch der Körper der Prothese in eine laterale zylindrische Portion *(Abb. 03/109)* und einen Schenkelhalsportion *(Abb. 03/110)* unterteilt wird, verbunden durch eine Verbindungsportion *(Abb. 03/111).* Die dorsale Einziehung im Querschnitt verläuft geradlinig über die Gesamtlänge des Stieles, nimmt jedoch an Tiefe ab *(Abb. 05/112),* die Querschnitte sind ungleichförmig nierenförmig *(Abb. 05/112).*

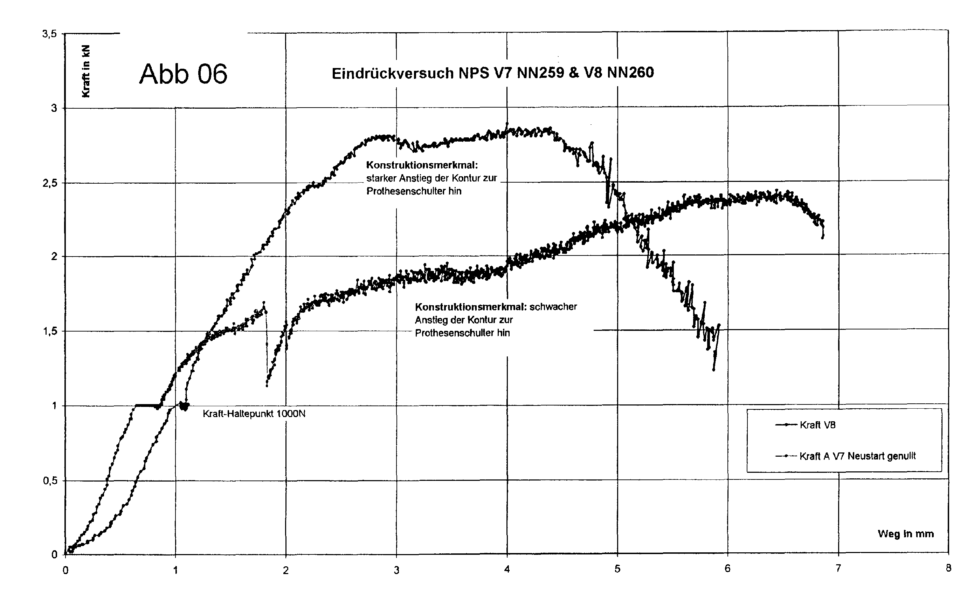

Die Mantelflächen sind darüber hinaus noch durch coaxial ausgerichtete Längsrillen und Rippen strukturiert, in der Regel ventral und dorsal. Die Kurvatur des Designs zeigt im U-Shape der Kraftübertragung *(Abb. 02.1/114)* eine parabelförmige Krümmung, welche die Knochenstruktur überlagert *(Abb. 02.1/115).* Die Mantelkrümmung bestimmt das Kraft/Weg-Diagramm im Belastungsversuch *(Abb. 06).*

In Verlängerung der Achse befindet sich das Einbringloch *(Abb. 04/201)* zum Ansetzen des Einschlaginstrumentes *(Abb. 04/140)* und eine korrespondierende Aufsatzfläche *(Abb. 04/202)* für den Aufschlag bzw. Gewinde bei den Probeprothesen für die Fixation des Einschlaginstrumentes *(Abb. 04/203).*

Beide Strukturen sind in der Schulter *(Abb. 04/200)* der Prothese untergebracht, die den Übergang zum Konus *(Abb. 04/300)* bildet, der den variablen Kopf *(Abb. 04/150)* trägt.

### Beispiel der Erfindung

Nach der üblichen Freilegung des Hüftgelenkes über den dorsalen oder auch seitlichen Zugang wird der Femurkopf entlang der geplanten Höhe unter Erhaltung des Schenkelhalses und Berücksichtigung der jeweiligen Metaphysenöffnungsebene abgesetzt.

Nach dem Einsetzen der Pfanne wird das Femur eingestellt, so dass die Markhöhle eröffnet werden kann.

In Verlängerung der Markkanalachse wird das Femur im Knie des großen Trochanters mit einer 11,2 mm im Durchmesser (Dm) messenden Diamanthohlschleife eröffnet und das gerade Führungsinstrument mit einem Dm von 11 mm und einer auf 6 mm verjüngter Spitze eingeführt *(Abb 05).*

Im Anschluss daran wird mit einer außen beschichteten Diamanthohlschleife die Metaphyse des Femur aufgeschliffen und eine Probeprothese an das Einschlaginstrument montiert *(Abb. 04).* Die Spitze der Probeprothese wird nun über das Eröffnungsloch eingefädelt und mit vorsichtigen Hammerschlägen in die Femurmetaphyse eingeschlagen. Ist dies nicht möglich, wird unter sorgfältiger Erhaltung der hinteren Wand des Schenkelhalses die Kanalrinne des Femurs nach dorsal ausgeschliffen, so dass die zylindrische Komponente des Schaftes sich hinter den Schenkelhals einfädelt.

Die Größe der Prothese wird nach 2 Maßen bestimmt: Das Führungsinstrument hat eine Längenmesskala, die die Prothesengrößen anzeigt. Das Meßinstrument muß in der S-förmigen Schwingung des Femur sicher verblockt sein, die Größe kann am Übergang des Schenkelhalses zum großen Trochanter abgelesen werden *(Abb. 06).* Mit einer Schublehre kann im sagittalen Strahlengang der größte Dm der inneren Kontur des Schenkelhalses ermittelt werden, die Schublehre zeigt ebenfalls die Prothesengröße an.

Die definitive Prothese wird in derselben Weise eingeschlagen, bis sie unverrückbar fest im Trichter des Schenkelhalses verankert ist.

Im Anschluß daran wird mit einem Probekopf reponiert und die Luxationstendenz ermittelt. Besteht keine Luxationsgefahr, kann das definitive Pfanneninsert und der definitive Kopf aufgesetzt werden, danach wird wieder reponiert, mit dem Bildwandler dokumentiert und die Wunde unter Refixation der Muskeln in Schichten verschlossen.
- ***Abb.01***: ap-Ansicht Schenkelhalserhaltende Prothese (NPS) mit folgenden Merkmalen:
- *Abb. 01/100*: anatomischer Stiel, rechts/links Symmetrie
- *Abb. 01/101*: Offset
- *Abb. 01/102*: CCD-Winkel 135°
- *Abb. 01/103*: Konusaufrichtung
- *Abb. 01/110*: Eröffnungskanal für den Diamant
- *Abb. 01/113*: koaxiale Längsstrukturen
- *Abb. 01/114*: U-shape der Kraftübertragung:
dorsomediale, mediale und anteromediale Fläche, parabelförmig gekrümmte Mantelfläche
- *Abb. 01/120*: Osteotomie
- *Abb. 01/130*: Femurkanalachse
- *Abb. 01/200*: Stiel-Hals-Kopf Übergang = Schulter
- *Abb. 01/300*: Konus
- ***Abb. 02.1***: axiale Ansicht einer NPS mit folgenden Merkmalen
- *Abb. 02.1/100*: Stiel
- *Abb. 02.1/104*: dorsale Rinne, Einziehung, axial bis zur Spitze des Stieles
- *Abb. 02.1/105*: dorsale, proximale Verblockung
- *Abb. 02.1/106*: ventrale Verblockung
- *Abb. 02.1/107*: dorsale, distale Verblockung
- *Abb. 02.1/105*: Verblockung des Schaftes im sagittalen Strahlengang
- *bis Abb. 02.1/107* *Abb. 02.1/114*: überragendes U-shape mit parabelförmiger Mantelkrümmung
- *Abb. 02.1/115*: unterragende Knochenstruktur entlang der Osteotomie
- *Abb. 02.1/200*: Schulter (Stiel-Hals-Konus) - Übergang
- *Abb. 02.1/300*: Konus
- ***Abb. 02.2***: Rechts/Links Spiegelsymmetrie
- ***Abb. 03***: Proximaler Querschnitt des Stieles mit
- *Abb. 03/108*: konvex-konkav-konvexe dorsale Krümmung
- *Abb. 03/109*: zylindrisch lateraler Portion
- *Abb. 03/110*: mediale Schenkelhalsportion
- *Abb. 03/111*: Übergangsstück
- ***Abb. 04***: NPS-Prothese mit
- *Abb. 04/140*: Applikationsinstrument
- *Abb. 04/150*: Kopfkugel
- *Abb. 04/200*: Schulterkrümmung, Schulterteil
- *Abb. 04/201*: Einbringloch
- *Abb. 04/202*: Aufsatzfläche, resp. Gewindebohrung (203) der Probeprothese
- *Abb. 04/203*: Gewindebohrung der Probeprothese
- *Abb. 04/300*: Konusmantelfläche, Konus
- ***Abb. 05***: NPS-Prothese
- *Abb. 05/100*: mit Stiel und
- *Abb. 05/112*: Querschnitten
- ***Abb. 06***: Kraft/Weg-Diagramm im Belastungsversuch beim NPS im humanen Femur mit zwei verschiedenen Mantelkrümmungen.

## Patentansprüche

1. Anatomische Femurkomponente eines künstlichen Hüftgelenkes für die zementfreie oder zementierte Implantation durch folgende Merkmale ausgezeichnet:
- der Prothesenstiel weist dorsal eine Einziehung auf, die axial geradlinig in die Spitze der Prothese ausläuft und proximal dorsal die Knochenstrukturen des Schenkelhalses vom Übergang in das Trochantermajormassiv aufnimmt;
- die dorsale konkave Einziehung unterteilt den Stiel proximal in zwei deutlich abgesetzte Portionen: eine mediale, proximal kegelförmig aufgetriebene Portion und eine laterale über die Länge des Stieles axial nahezu zylindrisch gestaltete Portion.
- der Querschnitt des Stieles ist proximal plump kommaförmig gestaltet.

2. Femurkomponente nach Anspruch (1) durch folgende Merkmale ausgezeichnet:
- der Prothesenstiel weist dorsal eine Einziehung auf, die axial geradlinig in die Spitze der Prothese ausläuft und proximal dorsal die Knochenstrukturen des Schenkelhalses vom Übergang in das Trochantermassiv aufnimmt.
- die dorsale konkave Einziehung unterteilt den Stiel proximal in zwei deutlich abgesetzte Portionen: eine mediale, proximal kegelförmig aufgetriebene Portion und eine laterale über die Länge des Stieles axial nahezu zylindrisch gestaltete Portion.
- der Prothesenstiel weist zusätzlich ventral eine Einziehung auf, die axial nahezu geradlinig in die Spitze der Prothese ausläuft und den Stiel in eine laterale fast zylindrische Portion und einen medialen, proximal kegelförmig aufgetriebenen Abschnitt unterteilt.

3. Femurkomponente eines künstlichen Hüftgelenkes für die zementfreie oder zementierte Implantation durch folgende Merkmale ausgezeichnet:
- der Prothesenstiel weist eine anatomisch konfigurierte kegelförmige, rechtslinks spiegelsymmetrisch ausgebildete mediale Portion und
- eine fest mit dieser verbundenen axial ausgerichtet mehr oder weniger zylindrischen Portion auf.

4. Femurkomponente nach Anspruch (1) bis (3) **dadurch gekennzeichnet, dass** der proximale Querschnitt sich kommaförmig mit Konkavität nach dorsal und punktförmiger Auftreibung nach medial darstellt und sich rechts und links spiegelsymmetrisch verhält.

5. Femurkomponente nach den Ansprüchen (1) bis (4) so konstruiert, dass die Femurstielachse mit der Femurkanalachse zusammenfällt und in der Frontalebene die Collum-Centrumsachse mit der Diaphysenachse (CCD-Winkel) einen Winkel zwischen 125° und 145°, in aller Regel 135°, einschließt und in der axialen Aufsicht einen Winkel zwischen Diaphyse und der Schenkelhalsachse, einen sogenannten Antetorsionswinkel zwischen 5° und 15°, in aller Regel 7°, umschließt.

6. Femurkomponente nach den Ansprüchen (1) bis (5) so gestaltet, dass die Ventralfläche axial konvex und nach ventral in der Weise konkav gekrümmt ist, dass der Krümmungsmittelpunkt ventral liegt und der Krümmungsradius sich nach proximal kontinuierlich verkleinert (Parabel).

7. Femurkomponente nach den Ansprüchen (1) bis (6) so konstruiert, dass die mediale Mantelfläche axial konvex und entlang der medialen Kontur konkav gekrümmt ist und zwar in der Weise, dass der Mantelkrümmungsmittelpunkt medial liegt und sein Radius sich nach proximal kontinuierlich verkleinert (Parabel).

8. Femurkomponente nach den Ansprüchen (1) bis (7) so konstruiert, dass die dorsale Mantelfläche des Stieles um die Achse herum proximal von lateral nach medial konvex-konkav-konvex gekrümmt ist in Form eines Wellenschlages oder einer gerundeten "3" mit asymmetrischen Schenkeln und gerundetem Übergang.

9. Femurkomponente nach den Ansprüchen (1) bis (8) so beschaffen, dass die laterale Mantelfläche weitgehend geradlinig, zylinderförmig konstruiert ist.

10. Femurkomponente nach den Ansprüchen (1) bis (9) so beschaffen, dass die Ventralfläche und/oder Medialfläche und/oder Dorsalfläche und/oder Lateralfläche durch coaxial ausgerichtete Längswülste strukturiert ist (sind).

11. Femurkomponente nach den Ansprüchen (1) bis (10) so konstruiert, dass der Stiel über eine Schulterkomponente (Kopf-Hals-Stiel-Übergang) in den Konus übergeht, der als modulares System verschiedene Köpfe zentrisch oder exzentrisch aufnehmen kann und der für die Aufnahme einer Zuggurtung eine zentrale Bohrung und/oder eine oder mehrere Bohrungen in der Schulter aufweist.

12. Femurkomponente nach den Ansprüchen (1) bis (11) so beschaffen, dass der Konus zwischen 2° und 9°, in aller Regel um 5°, in der Weise aufgerichtet ist, dass sich der CCD-Winkel nicht verändert und auch das Offset unverändert bleibt, die Achse des Konus sich jedoch in die laterodorsale Zirkumferenz des kompakten Femurs projiziert 2 cm- 4 cm unterhalb des Tuberculum innominatum.

13. Femurkomponente nach den Ansprüchen (1) bis (12) so beschaffen, dass das Implantat aus Titan, Tantal, CoCrMo oder einer Legierung aus Titan oder Tantal oder aus rostfreiem Stahl hergestellt ist.

14. Femurkomponente nach den Ansprüchen (1) bis (13) so beschaffen, dass die Oberfläche der proximalen Hälfte eine Rauhigkeit von 50-250 µm, vorzugsweise 80-100 um aufweist.

15. Femurkomponente nach den Ansprüchen (1) bis (14) so beschaffen, dass die Zuggurtung nach Anspruch (11), über die die Femurkomponente im Oberschenkelknochen unter Vorspannung im Schenkelhals verankert wird, als Schubstange ausgebildet ist, und zwar in der Form, dass sich die Schubstange in den Hüftkopf hineinschieben (Schubrichtung) kann, jedoch in Zugrichtung die Prothese vorspannt im Knochen.
